# EUROPEAN PATENT SPECIFICATION

(11) **EP 2 645 986 B1**
(45) Date of publication and mention of the grant of the patent: **25.02.2015**
(21) Application number: 11782644.6
(22) Date of filing: 17.11.2011
(51) Int. Cl.: A61K 8/36, A61K 8/46, A61K 8/49, A61Q 5/00, A61Q 5/02, A61K 31/555

(54) **ANTI-DANDRUFF SHAMPOO**
ANTISCHUPPENSHAMPOO
SHAMPOOING ANTIPELLICULAIRE

(30) Priority: 02.12.2010 EP 10193503; 02.12.2010 EP 10193505
(43) Date of publication of application: 09.10.2013
(73) Proprietor: Unilever PLC, London, Greater London EC4Y 0DY (GB); Unilever N.V., 3013 AL Rotterdam (NL)
(72) Inventor: HALL, Caroline, Alexandra, Wirral Merseyside CH63 3JW (GB); TURNER, Graham, Andrew, Wirral Merseyside CH63 3JW (GB); ZDRAVKOVA, Aneliya, Nikolova, Wirral Merseyside CH63 3JW (GB)
(74) Representative: Chisem, Janet
(86) International application number: PCT/EP2011/070388
(87) International publication number: WO 2012/072424

(56) References cited:
- EP-A1- 1 069 142
- WO-A1-97/26854
- US-A1- 2002 037 299
- Clariant: "Taking care of your customers' body: Hostapon", , May 2000 (2000-05), pages 1-7, XP002295661, Retrieved from the Internet: URL:http://fun.clariant/fun/internet.nsf/v wWebFramesets/723D035087361094C 1256CA800316780?openDocument [retrieved on 2004-09-09]
- Clariant: "Genapol LRO liquid", Innovadex , January 2010 (2010-01), page 1, XP002637790, Retrieved from the Internet: URL:http://www.innovadex.de/Cleaners/Detai l/2980/42455/GENAPOL-LRO-LIQUID [retrieved on 2011-05-19]
- Clariant: "Genagen CAB", Innovadex , August 2008 (2008-08), pages 1-2, XP002637791, Retrieved from the Internet: URL:http://www.innovadex.com/PersonalCare/ Detail/1022/42389/GENAGEN-CAB [retrieved on 2011-05-19]

## Description

The invention relates to an anti-dandruff shampoo composition with improved zinc deposition characteristics.

Dandruff is an issue that affects many people globally. The condition is manifested by the shedding of clumps of dead skin cells from the scalp. These are white in colour and provide an aesthetically displeasing appearance. A factor that contributes to dandruff are certain members of the *Malassezia* yeasts. To combat these, anti-dandruff products have included certain zinc salts which have anti-fungal activity, for example zinc pyrithione (ZnPTO). Such a product has to perform as a hair cleansing shampoo, while mitigating the causes of dandruff. An example of a known anti-dandruff shampoo comprises sodium lauryl ether sulfate (an ethoxylated anionic surfactant) in combination with zinc pyrithione.

There is a problem with such anti-dandruff shampoos in that deposition of the anti-dandruff zinc salt is not optimal.

It is an object of the invention to improve upon the anti-dandruff zinc salt deposition performance.

WO 97/26854 A1 discloses antidandruff shampoo compositions with alleged improved deposition of antimicrobial agents and which compositions comprise from about 7 percent to about 30 percent by weight of a detersive surfactant.

EP 1 069 142 A1 discloses water soluble polymers produced by copolymerization of macromonomers based on polyalkylene oxides.

US 2002/0037299 A discloses cosmetic preparations comprising the zinc salt of 1-hydroxy-4-ethyl-6-(2,4,4-trimethylpentyl)-2(1H)pyridone (Zn-Octopirox).

We have found that branched isethionate provides an improvement in the deposition of anti-dandruff zinc salts onto the scalp.

### Summary of the Invention

The invention thus provides in a first aspect an anti-dandruff shampoo comprising:-
a) from 0.1 to 5 wt.% of an anti-dandruff zinc salt;
b) from 1 to 8 wt.% of a branched alkyloyl isethionate;
c) from 1 to 10 wt.% of a fatty acyl isethionate product which product comprises 40 to 80 wt.% fatty acyl isethionate and 15 to 50 wt.% free fatty acid and/or fatty acid salt; and,
d) from 0.5 to 14 wt.% of a co-surfactant.

The anti-dandruff shampoo comprises an antidandruff zinc salt. The anti-dandruff zinc salts are preferably selected from zinc pyrithione, zinc sulfate and hydrates thereof (e.g. zinc sulfate hexahydrate), and combinations thereof. Zinc pyrithione (ZnPTO) shorthand for zinc 1-hydroxy-2-pyridinethione is most preferred.

The anti-dandruff zinc salt is present at a level of from 0.1 to 5 wt.%, preferably from 0.2 to 3 wt.%, more preferably from 0.25 to 2.5 wt.% based on the shampoo composition.

The anti-dandruff shampoo comprises from 1 to 8 wt.%, preferably from 2 to 7 wt.% of a branched alkyloyl isethionate.

Preferably the branched alkyloyl isethionate has the formula: RCOOCR¹HCR²HSO₃M; wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one of R, R¹ and R² are branched; and M is a solubilising cation such as sodium, potassium, ammonium or substituted ammonium.

A more preferred branched alkyloyl isethionate is shown in formula 1: 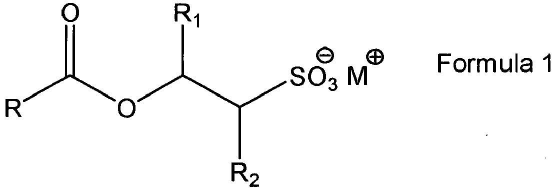 wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one R¹ and R² are selected from CH₃ or CH₂CH₃; and M is a solubilising cation such as sodium, potassium, ammonium or substituted ammonium.

Preferred branched alkyloyl isethionates are sodium fatty acid methyl isethionate, and sodium fatty acid ethyl isethionate, for example sodium cocoyl methyl isethionate or sodium cocoyl ethyl isethionate.

The fatty acyl isethionate product is present at a level of from 1 to 10 wt.%, preferably from 2 to 8 wt.%, more preferably from 2.5 to 7.5 wt.%.

The preferred fatty acyl isethionate product comprises fatty acyl isethionate surfactant at a level of from 40 to 80 wt.% of the product, as well as free fatty acid and/or fatty acid salt at a level of from 15 to 50%.

Preferably, greater than 20 wt.% and less than 45 wt. %, more preferably greater than 25 wt.% and less than 45 wt. % of the fatty acyl isethionate are of chain length greater than or equal to C₁₆; and greater than 50 wt.%, preferably greater than 60 wt.% of the free fatty acid/soap is of chain length C₁₆ to C₂₀.

The shampoo comprises from 0.5 to 14 wt.%, preferably from 1 to 12 wt.%, more preferably from 1.5 to 10 wt.% of a co-surfactant. Preferred co-surfactants are selected from anionic, nonionic, cationic, amphoteric surfactants, or mixtures thereof.

In a preferred embodiment, the anti-dandruff shampoo comprises an alkyl sulphate and/or ethoxylated alkyl sulfate anionic surfactant at a level of from 2 to 14 wt.%, preferably from 3 to 12 wt.%, more preferably from 4 to 10 wt.%.

Preferred alkyl sulfates are C₈₋₁₈ alky sulfates, more preferably C₁₂₋₁₈ alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES) having an average degree of ethoxylation of from 0.5 to 3, preferably 1 to 3.

In a preferred embodiment, the anti-dandruff shampoo comprises from 0.1 to 10 wt.%, preferably from 0.5 to 8 wt.%, more preferably from 1 to 5 wt.% of a betaine surfactant, preferably an alkyl amidopropyl betaine, for example cocamidopropyl betaine.

In a preferred embodiment, the anti-dandruff shampoo comprises from 0.05 to 1 wt.% of a C₈-C₁₆ fatty acid, preferably lauric acid.

A particularly preferred embodiment of the invention is an anti-dandruff shampoo comprising:-
a) from 0.25 to 2.5 wt.% of zinc pyrithione;
b) from 1 to 8 wt.% of a branched alkyloyl isethionate;
c) from 4 to 10 wt.% of sodium lauryl ether sulfate having an average degree of ethoxylation of from 0.5 to 3;
d) from 1 to 10 wt.% of a fatty acyl isethionate product which product comprises 40 to 80 wt.% fatty acyl isethionate and 15 to 50 wt.% free fatty acid and/or fatty acid salt; and,
e) from 0.1 to 5 wt.% of cocamidopropyl betaine.

A second aspect of the invention relates to the use of a branched alkyloyl isethionate to improve the deposition of an anti-dandruff zinc salt onto the scalp.

Preferably the branched alkyloyl isethionate has the structure: RCOOCR¹HCR²HSO₃M; wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one of R, R¹ and R² are branched; and M is a solubilising cation such as sodium, potassium, ammonium or substituted ammonium.

More preferably the branched alkyloyl isethionate comprises sodium fatty acid methyl isethionate or sodium fatty acid ethyl isethionate, wherein the fatty acid is a C₆-C₁₈ fatty acid, preferably derived from coconut oil.

A third aspect of the invention relations to a method of enhancing the deposition of an anti-dandruff zinc salt onto the scalp of a consumer, by treatment of the scalp with a shampoo comprising an anti-dandruff zinc salt, wherein the shampoo comprises a branched alkyloyl isethionate.

### Detailed Description of the Invention

### Fatty Acyl isethionate Product

The fatty acyl isethionate product is present at a level of from 1 to 10 wt.%, preferably from 2 to 8 wt.%, more preferably from 2.5 to 7.5 wt.%.

The preferred fatty acyl isethionate product comprises fatty acyl isethionate surfactant at a level of from 40 to 80 wt.% of the product, as well as free fatty acid and/or fatty acid salt at a level of from 15 to 50%.
Preferably, greater than 20 wt.% and less than 45 wt. %, more preferably greater than 25 wt.% and less than 45 wt. % of the fatty acyl isethionate are of chain length greater than or equal to C₁₆; and greater than 50 wt.%, preferably greater than 60 wt.% of the free fatty acid/soap is of chain length C₁₆ to C₂₀.

The fatty acyl isethionate surfactant component is typically prepared by the reaction of an isethionates salt such as alkali metal isethionates and an aliphatic fatty acid having 8 to 20 carbon atoms and Iodine Value (measuring degree of unsaturation) of less than 20 g, for example:

RCOOH + HOR₁SO₃M → RCOOR₁SO₃M

where R₁ is an aliphatic hydrocarbon radical containing 2 to 4 carbons; M is alkali metal cation or metal ion (e.g., sodium, magnesium, potassium, lithium), ammonium or substituted ammonium cation or other counterion; and, R is an aliphatic hydrocarbon radical having 7 to 24, preferably 8 to 22 carbons.

Depending on the processing conditions used, the resulting fatty acyl isethionate product can be a mixture of 40 to 80% by weight of fatty acyl isethionates (which formed from the reaction) and 50 to about 15 wt. %, typically 40 to 20 wt. % of free fatty acids. In addition, the product may contain isethionates salts which are present typically at levels less than 5 wt. %, and traces (less than 2 wt. %) of other impurities. Preferably, a mixture of aliphatic fatty acids is used for the preparation of commercial fatty acyl isethionates surfactants. The resulting fatty acyl isethionate surfactants (e.g., resulting from reaction of alkali metal isethionate and aliphatic fatty acid) preferably should have more than 20 wt. %, preferably more than 25 wt.%, but no more than 45 wt.%, preferably 35% (on basis of fatty acyl isethionates reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

Examples of commercial fatty acyl isethionate products that are particularly useful in the subject invention are DEFI flakes and Dove^{®} cleansing bar noodles produced by Unilever. DEFI (Direct Esterification of Fatty Isethionate) flakes typically contain about 68 to 80 wt. % of sodium fatty acyl isethionate and 15 to 30 wt. % free fatty acid. More than 25 wt. % and no more than 35% of fatty acyl group of the resulting fatty acyl isethionate have 16 to 18 carbon atoms. Dove^{®} cleansing bar noodles are mixtures of DEFI flakes described above and long chain (mainly C₁₆ and C₁₈) fatty acid and fatty soap which contain about 40 to 55 wt. % of fatty acyl isethionate and 30 to 40 wt. % of fatty acid and fatty soap.

### Zinc active

The anti-dandruff shampoo comprises an antidandruff zinc salt. The anti-dandruff zinc salts may be selected from zinc pyrithione, zinc sulfate and hydrates thereof (e.g. zinc sulfate hexahydrate), and combinations. Zinc pyrithione (ZnPTO) which is an alternate name for zinc 1-hydroxy-2-pyridinethione is preferred.

The anti-dandruff zinc salt is present at a level of from 0.1 to 5 wt.%, preferably from 0.2 to 3 wt.%, more preferably from 0.25 to 2.5 wt.% based on the anti-dandruff shampoo composition.

### Other AD actives

Additional anti-dandruff actives may be included in the compositions. Illustrative substances are octopirox (piroctone olamine), azole antimicrobials (e.g. climbazole), selenium sulfide and combinations thereof. Amounts of these materials may range from about 0.01 to about 5 wt.%, preferably from 0.1 to 3 wt.%, and optimally from about 0.3 to about 4 wt.% of the composition.

### Branched Alkyloyl Isethionate

The anti-dandruff shampoo comprises from 1 to 8 wt.%, preferably from 2 to 7 wt.% of a branched alkyloyl isethionate.

Preferably the branched alkyloyl isethionate has the formula: RCOOCR¹HCR²HSO₃M; wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one of R, R¹ and R² are branched; and M is a solubilising cation such as sodium, potassium, ammonium or substituted ammonium.

As the branched alkyloyl isethionate component is typically prepared by the reaction of an isethionates salt such as alkali metal isethionates and an aliphatic fatty acid having 8 to 20 carbon atoms, the branching can occur in the ester from either or both of the isethionate or the fatty acid original feedstocks of the ester. Preferably the branch is located on the isethionate part of the ester.

A more preferred branched alkyloyl isethionate is shown in formula 1: 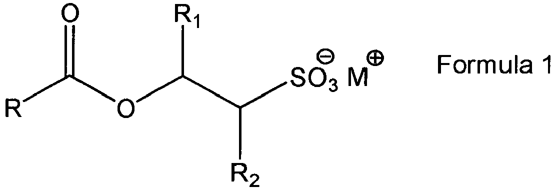 wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one of R¹ and R² are selected from CH₃ or CH₂CH₃; and M is a solubilising cation such as sodium, potassium, ammonium or substituted ammonium.

Preferred branched alkyloyl isethionates are sodium fatty acid methyl isethionate, and sodium fatty acid ethyl isethionate, for example sodium cocoyl methyl isethionate or sodium cocoyl ethyl isethionate.

### Co-surfactants

The shampoo comprises from 0.5 to 14 wt.%, preferably from 1 to 12 wt.%, more preferably from 1.5 to 10 wt.% of a co-surfactant. This co-surfactant is different to any other surfactant components of the claim. Preferred co-surfactants are selected from anionic, nonionic, cationic, amphoteric surfactants, or mixtures thereof.

### Anionic Cleansing Surfactant.

The anti-dandruff shampoo may comprise an anionic cleansing surfactant.

Preferred anionic cleansing surfactants are alkyl sulphate and/or ethoxylated alkyl sulfate anionic surfactant at a level of from 2 to 14 wt.%, preferably from 3 to 12 wt. %, more preferably from 1.5 to 10 wt. %.

Preferred alkyl sulfates are C₈₋₁₈ alky sulfates, more preferably C₁₂₋₁₈ alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3; and M is a solubilising cation such as sodium, potassium, ammonium or substituted ammonium. An example is sodium lauryl ether sulfate (SLES).

A preferred ethoxylated alkyl sulfate anionic surfactant is sodium lauryl ether sulfate (SLES) having an average degree of ethoxylation of from 0.5 to 3, preferably 1 to 3.

Shampoo compositions according to the invention may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

If added, the total amount of additional anionic cleansing surfactant in shampoo compositions of the invention may generally range from 0.5 to 45 wt.%, preferably from 1.5 to 35 wt.%, more preferably from 5 to 20 wt.%, calculated by total weight anionic cleansing surfactant based on the total weight of the composition.

The composition can include additional surfactants, to help impart aesthetic, physical or cleansing properties to the composition.

An example of an additional surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)n

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more; preferably, the value of n lies from about 1.1 to about 2; most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92/06154 and US 5,194, 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.1 to about 10 wt.%, preferably from 0.5 to 8, more preferably from 1 to 5 wt.%, based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 50%, preferably from 2 to 40%, more preferably from 10 to 25% by total weight surfactant based on the total weight of the composition.

### Fatty acid

The shampoo may additionally comprise from 0.05 to 1 wt.%, preferably from 0.075 to 0.5 wt.%, of a C₈-C₁₆ fatty acid.

The fatty acid is a C₈-C₁₆ fatty acid. The fatty acid may be a single fatty acid, or a mixture of fatty acids having a carbon number of from C₈-C₁₆. A preferred fatty acid is lauric acid. Lauric acid is a C₁₂ fatty acid.

### Silicone

Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Number average particle size diameters for the silicones may range from about 0.01 micron to about 50 micron, most preferably from about 0.01 to about 0.5 micron.

Advantageously the compositions of this invention may include a pre-mix of a silicone microemulsion. The microemulsion is an aqueous surfactant stabilized emulsion of silicone particles having a number average particle diameter ranging from about 10 to about 1,000 nm, preferably from about 100 to about 500 nm.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions or microemulsions of dimethiconol. Also suitable are amodimethicone emulsions such as DC939 (from Dow Corning) and SME253 (from GE Silicones).
Amounts of the silicone in compositions where present may range from about 0.01 to about 10 wt.%, preferably from about 0.1 to about 8wt.%, more preferably from about 0.3 to about 5wt.% by weight of the shampoo compositions.

### Cationic Polymer

A cationic polymer is a preferred ingredient in shampoo compositions according to the invention, for enhancing conditioning performance of the shampoo.

Preferably the anti-dandruff shampoo comprises from 0.1 to 5 wt.% of a cationic polymer, preferably a cationic polysaccharide polymer.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.
The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic conditioning polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic conditioning polymers can comprise mixtures of monomer units derived from amine- and/or quaternary ammonium-substituted monomer and/or compatible spacer monomers.

Suitable cationic conditioning polymers include, for example:
- copolymers of 1-vinyl-2-pyrrolidine and 1-vinyl-3-methyl-imidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic conditioning polymers that can be used include cationic polysaccharide polymers, such as cationic cellulose derivatives, cationic starch derivatives, and cationic guar gum derivatives. Suitably, such cationic polysaccharide polymers have a charge density in the range from 0.1 to 4 meq/g.

Cationic polysaccharide polymers suitable for use in compositions of the invention include those of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.
Preferably the cationic conditioning polymer is selected from cationic cellulose and cationic guar derivatives. Particularly preferred cationic polymers are JAGUAR C13S, JAGUAR C15, JAGUAR C17 and JAGUAR C16 and JAGUAR C162.

The cationic conditioning polymer will generally be present in compositions of the invention at levels of from 0.01 to 5, preferably from 0.05 to 1, more preferably from 0.08 to 0.5 percent by weight of the composition.

When cationic conditioning polymer is present in a shampoo composition according to the invention, it is preferred if the copolymer is present as emulsion particles with a mean diameter (D_{3,2} as measured by light scattering using a Malvern particle sizer) of 2 micrometres or less.

Shampoo compositions of the invention are preferably aqueous, i.e. they have water or an aqueous solution or a lyotropic liquid crystalline phase as their major component. Suitably, the composition will comprise from 50 to 98%, preferably from 60 to 90% water by weight based on the total weight of the composition.

### Suspending Agent

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent, if included, will generally be present in a shampoo composition of the invention at levels of from 0.1 to 10%, preferably from 0.5 to 6%, more preferably from 0.9 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The invention also relates to the use of a branched alkyloyl isethionate to enhance the deposition of an anti-dandruff zinc salt onto the scalp. Enhancement used in this context means that deposition of the zinc salt is increased by virtue of inclusion of the branched alkyloyl isethionate in the anti-dandruff shampoo formulation.

An alternative wording for this use is a method (or process) of enhancing the deposition of an anti-dandruff zinc salt onto the scalp from an anti-dandruff shampoo when used by a consumer, by virtue of inclusion of a branched alkyloyl isethionate in the anti-dandruff shampoo.

An embodiment of the invention is a method of enhancing the deposition of an anti-dandruff zinc salt (preferably zinc pyrithione) onto the scalp of a consumer, by treatment of the scalp with a shampoo comprising an anti-dandruff zinc salt (preferably zinc pyrithione) wherein the shampoo comprises a branched isethionate.

Preferably the branched alkyloyl isethionate has the structure: RCOOCR¹HCR²HSO₃M; wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one of R, R¹ and R² are branched; and M is a solubilising cation such as sodium, potassium, ammonium or substituted ammonium.

A highly preferred branched alkyloyl isethionate comprises sodium fatty acid methyl isethionate or sodium fatty acid ethyl isethionate, wherein the fatty acid is a C₆-C₁₈ fatty acid, preferably derived from coconut oil.

The invention will now be illustrated with reference to the following non-limiting Examples. Inventions according to the invention are denoted by a number, comparative inventions are denoted as such, or by using a letter.

### Examples

An improvement in terms of enhanced zinc deposition from an anti-dandruff shampoo by the incorporation of a branched alkyloyl isethionate is demonstrated. A further enhancement to this deposition by incorporation of from 0.05 to 1 wt.% of a C₈-C₁₆ fatty acid is also demonstrated.

Zinc deposition was measured by XRF (X-ray fluorescence spectroscopy). A combination of each shampoo (0.5 ml) and water (1.5ml) was applied to a 5cm by 5cm VitroSkin substrate for 30 seconds to mimic normal consumer use of shampoo onto the scalp. Application was followed by rinse-off, with the VitroSkin allowed to dry naturally overnight. The zinc deposition was then measured by XRF. Statistical analysis was by Student's t-test.

Vitro-Skin is an artificial substrate that is used in our deposition studies to mimic the surface properties of human skin. It contains both optimized protein and lipid components and is designed to have topography, pH, critical surface tension and ionic strength similar to human skin. Student's t-test is a well known statistical method that assesses whether the means of two groups are statistically different. The t-test compares the actual difference between two means in relation to the variation in the data.

### Example 1

The effect of the branched isethionate were tested in a model anti-dandruff shampoo base, containing (based on total wt.% of model shampoo ingredients & added ingredients in the test formulations):-6 wt.% sodium lauryl ether sulfate (SLES), 1.6 wt.% cocamidopropyl betaine (CAP-B), 0.2 wt.% cationic polymer (Jaguar C17), and, 1 wt.% zinc pyrithione, with the remainder minors and water. In this base shampoo, four different formulations were tested (a control without branched isethionate, and 3 with different branched isethionate levels), their additional components are shown in table 1:

**Table 1**

| **Ingredients added to base Anti-Dandruff shampoo** | **Comparative** | **Example 1** | **Example 2** | **Example 3** |
|---|---|---|---|---|
| Fatty Acyl Isethionate Product¹ | 8 | 6 | 4 | 2 |
| Branched isethionate² | - | 2 | 4 | 6 |

| | | | | |
|---|---|---|---|---|
| ¹ The Fatty Acyl Isethionate product is Sodium Cocoyl isethionate, Stearic Acid, Coconut Fatty Acid, Sodium Isethionate and Water produced in-house by Unilever ² The branched isethionate is sodium cocoyl methyl isethionate, available from Innospec | | | | |

The levels of extra ingredients (branched isethionate and fatty acyl isethionate product) were maintained at the same inclusion level in all four cases (8 wt.%). The levels of zinc deposition were measured and shown in table 2:

**Table 2 - Zinc Deposition Results (ppm)**

| | **Zinc deposition (ppm) (mean ± SD)** | **P value¹** |
|---|---|---|
| Comparative | 146 ± 6.8 | - |
| Example 1 | 172 ± 5.5 | 0.01 |
| Example 2 | 206 ± 14.6 | 0.002 |
| Example 3 | 244 ± 23.4 | 0.0008 |

| | | |
|---|---|---|
| ¹ p value is a measure of the confidence level that the statistical significance of the test result, in this case, the confidence is >99% for examples 1 to 3. | | |

The addition of branched isethionate to a formulation had a statistically significant (greater than 99% confidence level) improvement on the level of zinc deposition.

This is a single variable test and as such is clear experimental proof that addition of a branched alkyloyl isethionate results in an improvement in zinc deposition compared to a anti-dandruff shampoo without the branched alkyloyl isethionate.

### Example 2

The effect of lauric acid (an example of a C₈-C₁₆ fatty acid) on the deposition of zinc was tested by adding lauric acid to a model shampoo base. The formulations tested are shown in table 3 (ingredient inclusion is based on total wt.% of the shampoo):-

**Table 3**

| **Ingredients** | **Formulation A (wt.%)** | **Formulation B (wt.%)** | **Formulation C (wt.%)** |
|---|---|---|---|
| Fatty Acyl Isethionate Product¹ | 8 | 8 | 8 |
| SLES | 6 | 6 | 6 |
| Cocamidopropyl Betaine | 1.6 | 1.6 | 1.6 |
| Zinc Pyrithione | 1 | 1 | 1 |
| Lauric Acid² | - | 0.1 | 0.5 |
| Water | To 100 | To 100 | To 100 |

| | | | |
|---|---|---|---|
| ¹ The Fatty Acyl Isethionate product is Sodium Cocoyl Isethionate, Stearic Acid, Coconut Fatty Acid, Sodium Isethionate and Water produced in-house by Unilever ² Lauric Acid was sourced from Sigma | | | |

The levels of zinc deposition were measured and shown in table 4:

**Table 4 - Zinc Deposition Results (ppm)**

| | **Zinc Deposition (ppm) (Mean ± Standard Deviation)** |
|---|---|
| Formulation A | 146 ± 6.8 |
| Formulation B | 140 ± 6.0 |
| Formulation C | 159 ± 9.9 |

It is clear from this data that addition of lauric acid by itself provides no improvement to zinc deposition.

### Example 3

This example demonstrates that the improvement in zinc deposition seen for the branched alkyloyl isethionate in example 1 can be further enhanced by addition of a C₈-C₁₆ fatty acid.

In the base shampoo, two different formulations were tested, the first included 4 wt.% of a branched fatty acyl isethionate product; the second had a combination of both a branched fatty acyl isethionate product and lauric acid at a 0.5 wt.% inclusion levels. The additional components are shown in table 5:

**Table 5**

| **Ingredients added to the Base Anti-Dandruff shampoo** | **Comparative Formulation E** | **Invention Ex. 2** |
|---|---|---|
| Fatty Acyl Isethionate Product¹ | 4 | 4 |
| Branched Fatty Acyl Isethionate² | - | 4 |
| Lauric Acid | - | 0.5 |

| | | |
|---|---|---|
| ¹ The Fatty Acyl Isethionate product is Sodium Cocoyl isethionate, Stearic Acid, Coconut Fatty Acid, Sodium Isethionate and Water produced in-house by Unilever ² The Branched Fatty Acyl Isethionate is Sodium Cocoyl Methyl Isethionate | | |

The levels of zinc deposition were measured and shown in table 6:

**Table 6 - Zinc Deposition Results (ppm)**

| | **Zinc deposition (ppm) (Mean ± Standard Error)** | **P value¹** |
|---|---|---|
| Comparative E | 206 ± 8.5 | - |
| Example 2 | 252 ± 30.4 | p = 0.015 |

| | | |
|---|---|---|
| ¹ p value is a measure of the confidence level that the statistical significance of the test result, in this case, the confidence >98% for Example 2. | | |

Thus the combination of lauric acid and a branched isethionate provides a further improvement in zinc deposition.

### Example Formulations

| **Ingredient** | **Tradename** | **Ex I wt.%** | **Ex II wt.%** | **Ex III wt.%** | **Ex IV wt.%** |
|---|---|---|---|---|---|
| Sodium Laureth Sulfate | Texapon N70 | 4.0 | 6.0 | 2.0 | 4.0 |
| Cocamidopropyl Betaine | Tegobetaine CK | 2.0 | 1.6 | 3.0 | 2.0 |
| Fatty Acyl Isethionate Product¹ | | 2.0 | 3.0 | 6.0 | 5.0 |
| Sodium cocoyl glycinate | Hostapon SG | 3.0 | | | 3.0 |
| Sodium cocoyl methyl isethionate | Pureact SLMI-85 | 4 | 3 | 2 | 1.5 |
| Acrylates/Streareth-20 Methacrylate polymer | Aculyn 88 | 1.0 | 1.0 | 0.75 | 1.0 |
| Silicone Emulsion² | | 3.0 | 2.0 | 3.0 | 2.0 |
| Guar Hydroxypropyltrimonium Chloride | Cesmetic BF-7 | 0.25 | 0.25 | 0.25 | 0.25 |
| Zinc Pyrithione | Zinc Omadine FPS | 1.0 | 1.0 | 1.0 | 1.0 |
| Fragrance | | 0.75 | 0.75 | 0.75 | 0.75 |
| Aqua + minors | | to 100 | to 100 | to 100 | to 100 |

| | | | | | |
|---|---|---|---|---|---|
| ¹ The Fatty Acyl Isethionate product is Sodium Cocoyl isethionate, Stearic Acid, Coconut Fatty Acid, Sodium Isethionate and Water produced in-house by Unilever ² Mixture of silicone emulsions from Wacker and Dow | | | | | |

### Example Formulations

| **Ingredient** | **Tradename** | **Ex V wt.%** | **Ex VI wt.%** | **Ex VII wt.%** | **Ex VIII wt.%** |
|---|---|---|---|---|---|
| Sodium Laureth Sulfate | Texapon N70 | 4.0 | 6.0 | 2.0 | 4.0 |
| Cocamidopropyl Betaine | Tegobetaine CK | 2.0 | 1.6 | 3.0 | 2.0 |
| Fatty Acyl Isethionate Product¹ | | 2.0 | 3.0 | 6.0 | 5.0 |
| Sodium cocoyl glycinate | Hostapon SG | 3.0 | | | 3.0 |
| Sodium cocoyl methyl isethionate | Pureact SLMI-85 | 4 | 3 | 2 | 1.5 |
| Lauric Acid² | | 0.1 | 0.1 | 0.2 | 0.5 |
| Acrylates/Streareth-20 Methacrylate polymer | Aculyn 88 | 1.0 | 1.0 | 0.75 | 1.0 |
| Silicone Emulsion³ | | 3.0 | 2.0 | 3.0 | 2.0 |
| Guar Hydroxypropyltrimonium Chloride | Cesmetic BF-7 | 0.25 | 0.25 | 0.25 | 0.25 |
| Zinc Pyrithione | Zinc Omadine FPS | 1.0 | 1.0 | 1.0 | 1.0 |
| Fragrance | | 0.75 | 0.75 | 0.75 | 0.75 |
| Aqua + minors | | to 100 | to 100 | to 100 | to 100 |

| | | | | | |
|---|---|---|---|---|---|
| ¹ The Fatty Acyl Isethionate product is Sodium Cocoyl isethionate, Stearic Acid, Coconut Fatty Acid, Sodium Isethionate and Water produced in-house by Unilever ² Lauric Acid was sourced from Sigma ³ Mixture of silicone emulsions from Wacker and Dow | | | | | |

## Claims

1. An anti-dandruff shampoo comprising:-
a) from 0.1 to 5 wt.% of an anti-dandruff zinc salt;
b) from 1 to 8 wt.% of a branched alkyloyl isethionate;
c) from 1 to 10 wt.% of a fatty acyl isethionate product which product comprises 40 to 80 wt.% fatty acyl isethionate and 15 to 50 wt.% free fatty acid and/or fatty acid salt; and,
d) from 0.5 to 14 wt.% of a co-surfactant.

2. A shampoo according to claim 1, wherein the branched alkyloyl isethionate has the structure: RCOOCR¹HCR²HSO₃M; wherein R is a saturated or unsaturated alkyl or branched alkyl chain having from C₅₋₂₂ carbon atoms; R¹and R² are independently selected from H, or a branching group selected form CH₃ or CH₂CH₃, wherein at least one of R, R¹ and R² are branched; and M is a solubilising cation such as sodium, potassium, ammonium or substituted ammonium.

3. A shampoo according to claim 1 or claim 2, wherein the branched alkyloyl isethionate has a branch that is located on the isethionate part of the ester surfactant.

4. A shampoo according to any one of the preceding claims, wherein the branched alkyloyl isethionate comprises sodium fatty acid methyl isethionate or sodium fatty acid ethyl isethionate, wherein the fatty acid is a C₆-C₁₈ fatty acid, preferably derived from coconut oil.

5. A shampoo according to any one of the preceding claims, wherein the anti-dandruff zinc salt is zinc pyrithione (ZnPTO).

6. A shampoo according to any one of the preceding claims, wherein the anti-dandruff zinc salt is present at a level of from 0.25 to 2.5 wt.%.

7. A shampoo according to any one of the preceding claims, wherein the branched alkyloyl isethionate is present at a level of from 2 to 7 wt.%.

8. A shampoo according to any one of the preceding claims, wherein the co-surfactant comprises from 2 to 14 wt.% of an alkyl sulphate and/or and ethoxylated alkyl sulfate anionic surfactant.

9. A shampoo according to claim 8, wherein the alkyl sulfate and/or ethoxylated alkyl sulfate anionic surfactant comprises from 3 to 12 wt.%, preferably from 4 to 10 wt.% of sodium lauryl ether sulfate having an average degree of ethoxylation of from 0.5 to 3.

10. A shampoo according to any one of the preceding claims, additionally comprising from 0.1 to 10 wt.% of a betaine surfactant, preferably an alkyl amidopropyl betaine.

11. A shampoo according to any one of the preceding claims, additionally comprising from 0.05 to 1 wt.% of a C₈-C₁₆ fatty acid, preferably lauric acid.

12. Use of a branched alkyloyl isethionate to deposit an anti-dandruff zinc salt onto the scalp.

13. Use of a branched alkyloyl isethionate according to claim 12, wherein the branched alkyloyl isethionate is used in combination with a C₈-C₁₆ fatty acid to deposit an anti-dandruff zinc salt onto the scalp.

14. Use according to claim 12 or claim 13, wherein the branched alkyloyl isethionate comprises sodium fatty acid methyl isethionate or sodium fatty acid ethyl isethionate, wherein the fatty acid is lauric acid.

## Patentansprüche

1. Antischuppenshampoo,
das Folgendes aufweist:
a) 0,1 bis 5 Gew.-% eines gegen Schuppen gerichteten Zinksalzes,
b) 1 bis 8 Gew.-% eines verzweigten Alkyloylisethionats;
c) 1 bis 10 Gew.-% eines Fettacylisethionatproduktes, wobei das Produkt 40 bis 80 Gew.-% Fettacylisethionat und 15 bis 50 Gew.-% ungebundene Fettsäure und/oder ungebundenes Fettsäuresalz aufweist; und
d) 0,5 bis 14 Gew.-% eines Co-Tensids.

2. Shampoo nach Anspruch 1,
wobei das verzweigte Alkyloylisethionat die Struktur RCOOCR¹HCR²HSO₃M hat, worin R eine gesättigte oder ungesättigte Alkyl- oder verzweigte Alkylkette mit C₅₋₂₂-Kohlenstoffatomen ist, R¹ und R² unabhängig voneinander aus H oder einer Verzweigungsgruppe ausgewählt sind, die aus CH₃ oder CH₂CH₃ ausgewählt ist, wobei mindestens einer der Reste R, R¹ und R² verzweigt ist, und M ein löslichmachendes Kation, wie Natrium, Kalium, Ammonium oder substituiertes Ammonium, ist.

3. Shampoo nach Anspruch 1 oder 2,
wobei das verzweigte Alkyloylisethionat einer Verzweigung aufweist, die sich am Isethionatteil des Ester-Tensids befindet.

4. Shampoo nach einem der vorstehenden Ansprüche,
wobei das verzweigte Alkyloylisethionat Natriumfettsäuremethylisethionat oder Natriumfettsäureethylisethionat aufweist, wobei die Fettsäure eine C₆-C₁₈-Fettsäure ist, die vorzugsweise von Kokosnussöl abgeleitet ist.

5. Shampoo nach einem der vorstehenden Ansprüche,
wobei das gegen Schuppen gerichtete Zinksalz Zinkpyrithion (ZnPTO) ist.

6. Shampoo nach einem der vorstehenden Ansprüche,
wobei das gegen Schuppen gerichtete Zinksalz in einer Menge von 0,25 bis 2,5 Gew.-% vorliegt.

7. Shampoo nach einem der vorstehenden Ansprüche,
wobei das verzweigte Alkyloylisethionat in einer Menge von 2 bis 7 Ges.-% vorliegt.

8. Shampoo nach einem der vorstehenden Ansprüche,
wobei das Co-Tensid 2 bis 14 Gew.-% eines anionischen Tensids in Form von Alkylsulfat und/oder ethoxyliertem Alkylsulfat aufweist.

9. Shampoo nach Anspruch 8,
wobei das anionische Tensid in Form von Alkylsulfat und/oder ethoxyliertem Alkylsulfat 3 bis 12 Gew.-%, vorzugsweise 4 bis 10 Gew.-% Natriumlaurylethersulfat mit einem durchschnittlichen Ethoxylierungsgrad von 0,5 bis 3 aufweist.

10. Shampoo nach einem der vorstehenden Ansprüche,
das ferner 0,1 bis 10 Gew.-% Betain-Tensid, vorzugsweise Alkylamidopropylbetain, aufweist.

11. Shampoo nach einem der vorstehenden Ansprüche,
das ferner 0,05 bis 1 Gew.-% einer C₈-C₁₆-Fettsäure, vorzugsweise Laurinsäure, aufweist.

12. Verwendung eines verzweigten Alkyloylisethionats, um ein gegen Schuppen gerichtetes Zinksalz auf der Kopfhaut abzulagern.

13. Verwendung eines verzweigten Alkyloylisethionats nach Anspruch 12,
wobei das verzweigte Alkyloylisethionat in Kombination mit einer C₈-C₁₆-Fettsäure verwendet wird, um ein gegen Schuppen gerichtetes Zinksalz auf der Kopfhaut abzulagern.

14. Verwendung nach Anspruch 12 oder 13,
wobei das verzweigte Alkyloylisethionat Natriumfettsäuremethylisethonat oder Natriumfettsäureethylisethionat aufweist, wobei die Fettsäure Laurinsäure ist.

## Revendications

1. Shampooing antipelliculaire comprenant :
a) de 0,1 à 5 % en poids d'un sel de zinc antipelliculaire ;
b) de 1 à 8 % en poids d'un iséthionate d'alkyloyle ramifié ;
c) de 1 à 10 % en poids d'un produit à base d'iséthionate d'acyle d'acide gras, lequel produit comprend de 40 à 80 % en poids d'iséthionate d'acyle d'acide gras et de 15 à 50 % en poids d'un acide gras libre et/ou d'un sel d'acide gras ; et
d) de 0,5 à 14 % en poids d'un co-tensioactif.

2. Shampooing selon la revendication 1, dans lequel l'iséthionate d'alkyloyle ramifié a la structure : RCOOCR¹HCR²HSO₃M ; dans laquelle R est une chaîne alkyle ou alkyle ramifiée saturée ou non saturée ayant de 5 à 22 atomes de carbone ; R¹ et R² sont indépendamment choisis parmi H ou un groupe ramifiant choisi parmi CH₃ ou CH₂CH₃ où au moins un de R, R¹ et R² sont ramifiés ; et M est un cation de solubilisation tel que le sodium, le potassium, l'ammonium ou l'ammonium substitué.

3. Shampooing selon la revendication 1 ou la revendication 2, dans lequel l'iséthionate d'alkyloyle ramifié a une ramification qui est située sur la partie iséthionate du tensioactif de type ester.

4. Shampooing selon l'une quelconque des revendications précédentes, dans lequel l'iséthionate d'alkyloyle ramifié comprend l'iséthionate de méthyle d'acide gras ou l'iséthionate d'éthyle d'acide gras sodique, dans lequel l'acide gras est un acide gras en C₆ à C₁₈, de préférence dérivé de l'huile de coco.

5. Shampooing selon l'une quelconque des revendications précédentes, dans lequel le sel de zinc antipelliculaire est la pyrithione de zinc (ZnPTO).

6. Shampooing selon l'une quelconque des revendications précédentes, dans lequel le sel de zinc antipelliculaire est présent à un taux de 0,25 à 2,5 % en poids.

7. Shampooing selon l'une quelconque des revendications précédentes, dans lequel l'iséthionate d'alkyloyle ramifié est présent à un taux de 2 à 7 % en poids.

8. Shampooing selon l'une quelconque des revendications précédentes, dans lequel le co-tensioactif comprend de 2 à 14 % en poids d'un tensioactif anionique de sulfate d'alkyle et/ou de sulfate d'alkyle éthoxylé.

9. Shampooing selon la revendication 8, dans lequel le tensioactif anionique de sulfate d'alkyle et/ou de sulfate d'alkyle éthoxylé comprend de 3 à 12 % en poids, de préférence de 4 à 10 % en poids de sulfate d'éther laurylique sodique ayant un degré moyen d'éthoxylation de 0,5 à 3.

10. Shampooing selon l'une quelconque des revendications précédentes, comprenant en outre de 0,1 à 10 % en poids d'un tensioactif de bétaïne, de préférence une alkylamidopropylbétaïne.

11. Shampooing selon l'une quelconque des revendications précédentes, comprenant en outre de 0,05 à 1 % en poids d'un acide gras en C₈ à C₁₆, de préférence l'acide laurique.

12. Utilisation d'un iséthionate d'alkyloyle ramifié pour déposer un sel de zinc antipelliculaire sur le cuir chevelu.

13. Utilisation d'un iséthionate d'alkyloyle ramifié selon la revendication 12, dans laquelle l'iséthionate d'alkyloyle ramifié est utilisé en combinaison avec un acide gras en C₈ à C₁₆ pour déposer un sel de zinc antipelliculaire sur le cuir chevelu.

14. Utilisation selon la revendication 12 ou la revendication 13, dans laquelle l'iséthionate d'alkyloyle ramifié comprend l'iséthionate de méthyle d'acide gras ou l'iséthionate d'éthyle d'acide gras sodique, dans lequel l'acide gras est l'acide laurique.
